# DEMANDE DE BREVET EUROPEEN

(11) **EP 4 793 209 A1**
(43) Date de publication de la demande: **19.08.2026**
(21) Numéro de dépôt: 25226281.1
(22) Date de dépôt: 22.12.2025
(51) Int. Cl.: B65G 33/02, B65G 33/26

(54) **VIS D ALIMENTATION, DE MISE AU PAS ET/OU D ORIENTATION DE RÉCIPIENTS DANS UNE INSTALLATION DE CONDITIONNEMENT**

(30) Priorité: 13.02.2025 FR 2501503
(71) Demandeur: Sidel Participations, 76930 Octeville-sur-Mer (FR)
(72) Inventeur: DURIGON, Julien, 21910 CORCELLES-LES-CITEAUX (FR); BURDY, Théo, 21910 CORCELLES-LES-CITEAUX (FR)
(74) Mandataire: Sidel Group

(57) **Abrégé**

La présente invention concerne une unité (100) pour vis (1) d'alimentation, de mise au pas et/ou d' orientation de récipients (2) comprenant :
- un corps (3) central s'étendant depuis une extrémité amont (300) vers une extrémité aval (301) le long d'un axe longitudinal (X),
- un filet (4) de vis sensiblement hélicoïdal solidaire dudit corps (3), ledit filet (4) comprenant une pluralité d'hélices (40), chaque hélice (40) comprenant une crête (41), un flanc amont (42) et un flanc aval (43), ledit filet (4) étant configuré pour venir en prise entre lesdits récipients (2),
unité (100) caractérisée en ce qu'elle comprend pour chaque hélice (40) au moins :
- une première gorge (5a) sensiblement hélicoïdale au niveau du flanc amont (42) de ladite hélice (40) et apte à recevoir un premier jonc (50a), et
- une deuxième gorge (5b) sensiblement hélicoïdale au niveau du flanc aval (43) de ladite hélice (40) et apte à recevoir un deuxième jonc (50b).

L'invention concerne également une vis (1) d'alimentation, une installation (10) de traitement de récipients (2) et un procédé d'alimentation d'une entrée (70) d'une machine (7) de traitement.

## Description

### Domaine Technique

Dans le domaine du traitement de récipients, en particulier dans les installations de fabrication et de conditionnement de boissons, il est nécessaire d'alimenter chaque poste de traitement en récipients, de manière ordonnée, et selon une cadence prédéfinie.

Dans le cadre de l'invention, les récipients sont par exemple et de façon non limitative des bouteilles, des cannettes, des flacons, des bidons ou encore des briques cartonnées. Un tel récipients est destiné à contenir, de façon non exhaustive, un fluide, un liquide, des poudres ou des granulés, notamment de type agroalimentaire ou cosmétique.

Ces récipients peuvent être en tout type de matériau, en métal, en verre, mais préférentiellement à base de plastique, notamment en polyéthylène (PE) ou polypropylène (PP) conférant une souplesse rendant semi-rigide ledit récipients.

De tels récipients peuvent présenter tout type de forme, symétrique ou non, régulière ou irrégulière. De plus, chacun des récipients peut avoir une section arrondie, globalement de forme circulaire ou ovoïdale, ou bien une section polygonale, notamment rectangle ou carrée.

En particulier, l'utilisation d'un récipients de forme spécifique, par exemple asymétrique ou oblongue, est largement répandue dans le domaine des récipients alimentaires, des récipients d'entretien et des cosmétiques (FHPC pour « Food, Home care and Personal Care »), par exemple pour un récipient destiné à contenir un gel douche ou du shampooing, ou encore dans le domaine du nettoyage et de l'entretien, comme un flacon avec un pulvérisateur pour des détergents et désinfectants.

De manière connue, lesdits récipients sont déplacés le long de ladite ligne de production, à l'unité ou bien regroupés en lots, pour être acheminés vers différents postes de traitements successifs différents, comme la fabrication du contenant, par exemple lors d'une opération d'injection plastique ou d'étirage-soufflage dans le cas d'une bouteille en matériau plastique, suivi du remplissage puis de la fermeture par un bouchon et l'étiquetage. A l'issue de ces traitements, les récipients sont dits "finis".

En vue de leur manutention, de tels récipients finis subissent un conditionnement en lot. Chaque lot comprend un groupe de plusieurs récipients, rassemblés selon une rangée ou bien selon une disposition en matrice, généralement de forme globalement parallélépipédique, souvent carrée ou rectangle, selon plusieurs rangées et colonnes. A titre d'exemple, un lot habituel regroupe six récipients selon deux rangées et trois colonnes, souvent désigné « pack ».

Une fois les groupes de récipients réalisés, les groupes subissent généralement un conditionnement par encaissage ou par enrobage, notamment au travers d'une étape de filmage ou préférentiellement de fardelage, ou bien par un enrobage au moyen d'une feuille, notamment en matériau papier ou cartonné, afin de maintenir les récipients d'un même lot ensemble.

Au cours de ces différentes étapes, les récipients sont donc transportés le long de la ligne de production, selon une direction de déplacement s'étendant longitudinalement, depuis l'amont vers l'aval, entre et au sein des différents postes dédiés à chaque traitement que doivent subir les récipients.

En particulier, les récipients doivent être acheminés vers une machine de traitement via une entrée de récipient ou une alimentation de récipient, de telle manière que la distance entre deux récipients successifs corresponde à la cadence de la machine, c'est-à-dire à sa vitesse de traitement. Aussi, il peut être requis une modification de l'intervalle d'espacement des récipients successifs lors du passage d'un poste de traitement à un poste de traitement suivant.

En outre, il peut être nécessaire de modifier l'orientation des récipients avant l'entrée dans une machine de traitement, par exemple avant son entrée dans une remplisseuse ou étiqueteuse.

### Etat de la technique

Pour l'alimentation ordonnée et pour espacer ou séparer les récipients à un pas déterminé, c'est-à-dire à la distance correspondant à la cadence respective de la machine, il est connu d'avoir recours à un système à vis d'alimentation apte à pivoter sur elle-même et permettant par exemple d'augmenter le pas entre deux récipients alimentés successivement.

Il est également connu d'utiliser une telle vis pour la séparation des récipients, disposée du côté d'un itinéraire de transport pour les récipients et qui peut donc être entraînée en rotation.

Un des problèmes de ce type de vis d'alimentation est qu'elle est susceptible de laisser des marques de rayures sur le corps du récipient ainsi transporté.

En effet, une vis d'alimentation comprend un filetage ou filet, le bord de crête du filet entrant en contact avec les récipients à alimenter, mettre au pas et/ou orienter, ce contact créant alors des frictions.

L'invention propose de manière avantageuse une unité pour vis d'alimentation et vis d'alimentation, dont le filet hélicoïdal comprend au moins deux gorges, chacune des au moins deux gorges étant configurée pour recevoir un jonc. La présence du jonc permet d'éviter tout contact direct entre le récipient et le filet ou encore avec le corps de la vis. Le récipient étant en contact avec le jonc, le frottement est réduit et l'apparence ou l'intégrité dudit récipient n'est pas dégradée.

L'invention vise d'abord une unité pour vis d'alimentation, de mise au pas et/ou d'orientation de récipients comprenant :
- un corps central s'étendant depuis une extrémité amont vers une extrémité aval le long d'un axe longitudinal,
- un filet de vis sensiblement hélicoïdal solidaire dudit corps, ledit filet comprenant une pluralité d'hélices, chaque hélice comprenant une crête, un flanc amont et un flanc aval, ledit filet étant configuré pour venir en prise entre lesdits récipients.

L'unité selon l'invention est caractérisée en ce qu'elle comprend pour chaque hélice au moins :
- une première gorge sensiblement hélicoïdale au niveau du flanc amont de ladite hélice et apte à recevoir un premier jonc et
- une deuxième gorge sensiblement hélicoïdale au niveau du flanc aval de ladite hélice et apte à recevoir un deuxième jonc.

Dans des modes de réalisation, l'unité pour vis d'alimentation comprend une troisième gorge sensiblement hélicoïdale au niveau du flanc aval de ladite hélice et apte à recevoir un troisième jonc.

Dans des modes de réalisation, l'unité pour vis est réalisée d'un seul tenant.

Dans des modes de réalisation possible, l'unité pour vis est réalisée en impression 3D, et de préférence en polyamide.

Dans des modes de réalisation, les au moins deux gorges ont le même profil hélicoïdal que l'hélice.

Selon une caractéristique additionnelle possible, dans un plan axial perpendiculaire à l'axe longitudinal du corps de l'unité pour vis, l'angle amont du flanc amont de l'hélice est identique à l'angle aval du flanc aval de ladite hélice.

L'invention vise également une vis d'alimentation, de mise au pas et/ou d'orientation de récipients, ladite vis s'étendant depuis une extrémité amont vers une extrémité aval.

La vis selon l'invention est caractérisée en ce qu'elle comprend :
- un axe central,
- au moins une unité telle que décrite ci-dessus, ledit axe étant inséré dans le corps de ladite unité.

Dans des modes de réalisation préférés, la vis comprend au moins deux unités, lesdites unités étant aboutées les unes aux autres au moyen dudit axe central inséré dans le corps de chacune desdites au moins deux unités, de sorte que les au moins deux gorges de chacune desdites au moins deux unités s'étendent de manière continue depuis l'extrémité amont vers l'extrémité aval.

Selon une caractéristique additionnelle possible, les au moins deux gorges comprennent au moins un point de fixation pour jonc.

Dans des modes de réalisation, la vis comprend au moins deux joncs, lesdits au moins deux joncs ayant une forme complémentaire à la forme de la gorge respective et s'étendant sur toute la longueur de celle-ci.

Selon une caractéristique additionnelle possible, les au moins deux joncs sont en polymère, de préférence en Polytétrafluoroéthylène.

Dans des modes de réalisation, depuis l'extrémité amont et jusqu'à l'extrémité aval, les hélices formant le filet ont un rayon décroissant et le pas séparant deux hélices successives est décroissant.

L'invention vise encore une installation de traitement de récipients comprenant au moins :
- un dispositif de convoyage desdits récipients,
- une entrée d'une machine de traitement,
- au moins un arbre rotatif.

L'installation selon l'invention est caractérisée en ce qu'elle comprend au moins une vis d'alimentation, de mise au pas et/ou d'orientation telle que décrite précédemment, ladite vis étant disposée horizontalement et s'étendant de l'amont vers l'aval selon une direction le long d'un côté dudit dispositif de convoyage, ladite au moins une vise étant fixée sur ledit au moins un arbre rotatif afin de permettre sa mise en rotation.

Selon une caractéristique additionnelle possible, l'installation comprend en outre un rail de guidage situé du côté opposé et le long du dispositif de convoyage, ledit rail faisant face à ladite vis.

Selon un autre mode de réalisation, l'installation comprend un deuxième arbre rotatif et une deuxième vis, ladite deuxième vis étant situé du côté opposé du dispositif de convoyage et s'étendant depuis l'amont vers l'aval selon la direction, ladite deuxième vis étant fixée sur ledit deuxième arbre rotatif relié à ladite deuxième vis afin de permettre sa mise en rotation, les deux vis étant symétriques et configurées pour tourner dans un sens opposé.

L'invention a enfin pour objet un procédé d'alimentation en récipients d'une entrée d'une machine de traitement desdits récipients, ledit procédé comprenant au moins les étapes suivantes :
- transporter les récipients sur un dispositif de convoyage,
- mettre au pas et/ou orienter lesdits récipients avant leur entrée dans la machine de traitement, chaque corps du récipient étant apte à s'engager au niveau de l'extrémité amont d'une vis entre deux hélices formées sur le pourtour du corps de ladite vis.

Le procédé selon l'invention est caractérisé en ce que l'étape de mise au pas et/ou d'orientation des récipients est mise en œuvre au moyen d'au moins une vis telle que décrite précédemment.

**Brève description des figures** : L'invention sera mieux comprise grâce à la description ci-dessous, qui se base sur des modes de réalisations possibles, expliqués de façon illustrative et nullement limitative, en référence avec les figures annexées, dans lesquelles :
- [Fig. 1] représente schématiquement une vue du dessus d'un mode de réalisation possible d'une installation de conditionnement, les récipients étant des bouteilles ;
- [Fig.2] représente schématiquement une vue du dessus un mode de réalisation possible d'une installation de conditionnement, les récipients étant des flacons de forme sensiblement rectangulaire ;
- [Fig.3] représente schématiquement une vue en perspective d'un mode de réalisation possible d'une vis d'alimentation comprenant plusieurs unités ;
- [Fig.4] représente schématiquement un mode de réalisation possible d'une vis d'alimentation comprenant une pluralité d'hélices, avec un pas et un diamètre constants ;
- [Fig.5] représente schématiquement un mode de réalisation possible d'une vis d'alimentation comprenant une pluralité d'hélices, avec un pas et un diamètre décroissants ;
- [Fig.6] représente schématiquement un mode de réalisation possible d'une unité pour vis d'alimentation, comprenant deux gorges ;
- [Fig.7] représente schématiquement un autre mode de réalisation possible d'une unité pour vis d'alimentation, comprenant deux gorges ;
- [Fig.8] représente schématiquement un mode de réalisation possible d'une unité pour vis d'alimentation, comprenant trois gorges ;
- [Fig.9] représente schématiquement une vue de côté d'un mode de réalisation possible d'une unité pour vis d'alimentation, comprenant trois gorges et
- [Fig.10] représente une vue en coupe d'un mode de réalisation possible d'une unité pour vis d'alimentation.

**Description détaillée** : Dans la suite de la description, des éléments présentant une structure identique ou des fonctions analogues seront désignés par une même référence. L'invention vise tout d'abord une unité 100 pour vis 1 d'alimentation, de mise au pas et/ou d'orientation, configurée pour être mise en œuvre dans une installation de conditionnement de récipients 2.

Dans le cadre de l'invention, la vis 1 d'alimentation, de mise au pas et/ou d'orientation, ci-après dite vis 1 d'alimentation, est destinée à l'alimentation en récipients 2 d'une machine 7 de traitement. Elle peut également, dans des modes de réalisation, permettre la mise au pas ou la séparation de récipients 2, c'est-à-dire que la vis 1 permet de définir une distance ou intervalle précis qui sépare deux récipients 1 successifs, lesdits récipients 1 circulant sur un dispositif 6 de convoyage. Cela permet de définir une distribution ou alimentation exacte en fonction des besoins de la machine 7 de traitement.

Enfin, la vis 1 peut modifier l'orientation des récipients 2 circulant avant leur entrée dans la machine 7 de traitement.

La vis 1 agit de manière similaire à une came cylindrique et peut être appelée « vis sans fin ». En outre, la vis 1 est destinée à être mise en œuvre au niveau du dispositif 6 de convoyage sur lequel circulent des récipients 2.

Les récipients 2 se présentent les uns derrière les autres sur le dispositif 6 de convoyage, et pénètrent de façon continue dans le filet 4 de la vis 1. Lors de cette prise des récipients 2 dans le filet 4, une certaine friction est provoquée, pouvant détériorer le corps 20 desdits récipients 2.

Les récipients 2 sont par exemple et de façon non limitative des bouteilles, des cannettes, des flacons, des bidons ou encore des briques cartonnées. Un tel récipient 2 est destiné à contenir, de façon non exhaustive, un fluide, un liquide, des poudres ou des granulés, notamment de type agroalimentaire ou cosmétique.

Ces récipients 2 peuvent être en tout type de matériau, en métal, en verre, mais préférentiellement à base de plastique, notamment en polyéthylène (PE) ou polypropylène (PP) conférant une souplesse rendant semi-rigide ledit récipient 2.

De tels récipients 2 peuvent présenter tout type de forme, symétrique ou non, régulière ou irrégulière. De plus, chacun des récipients 2 peut avoir une section arrondie, globalement de forme circulaire ou ovoïdale, ou bien une section polygonale, notamment rectangle ou carrée.

En particulier, l'utilisation d'un récipient 2 de forme spécifique, par exemple asymétrique ou oblongue, est largement répandue dans le domaine des récipients alimentaires, des récipients d'entretien et des cosmétiques (FHPC pour « Food, Home care and Personal Care »), par exemple pour un récipient 2 destiné à contenir un gel douche ou du shampooing, ou encore dans le domaine du nettoyage et de l'entretien, comme un flacon avec un pulvérisateur pour des détergents et désinfectants.

Dans le cadre de l'invention, l'unité 100 pour vis 1 d'alimentation comprend :
- un corps 3 central, ou noyau, avec une extrémité amont 300 et une extrémité aval 301 le long d'un axe longitudinal X,
- un filet 4 de vis sensiblement hélicoïdal et solidaire dudit corps 3, ledit filet 4 comprenant une pluralité d'hélices 40, chaque hélice 40 comprenant une crête 41, un flanc amont 42 et un flanc aval 43, ledit filet 4 étant configuré pour venir en prise entre lesdits récipients 2.

En outre, l'axe de chaque hélice 40 est parallèle à l'axe longitudinal X du corps 3 central. Le corps 3 central est creux, c'est-à-dire qu'il comprend une ouverture, cavité ou encore orifice, s'étendant depuis l'extrémité amont 300 jusqu'à l'extrémité aval 301.

En d'autres termes, une unité 100 comprend au moins deux hélices 40 ou encore cannelures avec un profil hélicoïdal.

L'unité 100 est configurée de telle manière que les récipients 2 entrent dans le filet 4 au niveau de l'espace 44 ou creux séparant deux hélices 40 successives afin d'être pris en charge successivement par l'unité 100.

L'unité 100 se caractérise en ce qu'elle comprend pour chaque hélice 40 au moins :
- une première gorge 5a sensiblement hélicoïdale au niveau du flanc amont 42 de ladite hélice 40, ladite première gorge 5a étant apte à recevoir un premier jonc 50a,
- une deuxième gorge 5b sensiblement hélicoïdale au niveau du flanc aval 43 de ladite hélice 40, ladite deuxième gorge étant apte à recevoir un deuxième jonc 5b.

La première gorge 5a est présente au niveau du flanc amont 42 de l'hélice 40, ledit flanc amont 42 étant situé du côté de l'extrémité amont 300 le long de l'axe longitudinal X. En d'autres termes, le flanc amont 42 comprend au moins une gorge 5a sensiblement hélicoïdale. De préférence, la gorge 5a est continue sur la révolution de l'unité 100 et de faible profondeur. Par exemple, la profondeur de la gorge 5a est d'environ six millimètres (mm).

Dans un mode de réalisation préféré, la gorge 5a se situe au niveau de la partie supérieure du flanc amont 42, c'est-à-dire que la gorge 5a est située à proximité de la crête 41.

De préférence, la position de la au moins une gorge 5a est déterminée en fonction de la forme et de la taille des récipients 2 à traiter.

Dans des modes de réalisation, le nombre de gorges et la position de ces dernières au niveau du flanc amont 42 est déterminée en fonction de la forme et de la taille des récipients 2 à traiter.

La deuxième gorge 5b est présente au niveau du flanc aval 43, ledit flanc aval 43 étant située du côté de l'extrémité aval 301 le long de l'axe longitudinal X. En d'autres termes, le flanc aval 43 comprend au moins une gorge 5b sensiblement hélicoïdale.

De préférence, la gorge 5b est continue sur la révolution de l'unité 100 et de faible profondeur. Par exemple, la profondeur de la gorge 5b est d'environ six millimètres (mm). Dans des modes de réalisation, la gorge 5b se situe au niveau de la partie supérieure du flanc aval 43, c'est-à-dire que la gorge 5b est située à proximité de la crête 41.

Dans des modes de réalisation, la gorge 5b se situe au niveau de la partie inférieure du flanc aval 43, c'est-à-dire que la gorge 5b est située à proximité du creux ou espace 44 entre deux hélices 40.

De préférence, la position de la au moins une gorge 5b est déterminée en fonction de la forme et de la taille des récipients 2 à traiter.

Dans des modes de réalisation, le nombre de gorges et la position de ces dernières au niveau du flanc aval 43 est déterminée en fonction de la forme et de la taille des récipients 2 à traiter.

En d'autres termes, en fonction de la forme du récipient 2, mais aussi de ses dimensions, chacune des au moins deux gorges 5a, 5b est disposée à une distance prédéfinie de la crête 41 de l'hélice 40.

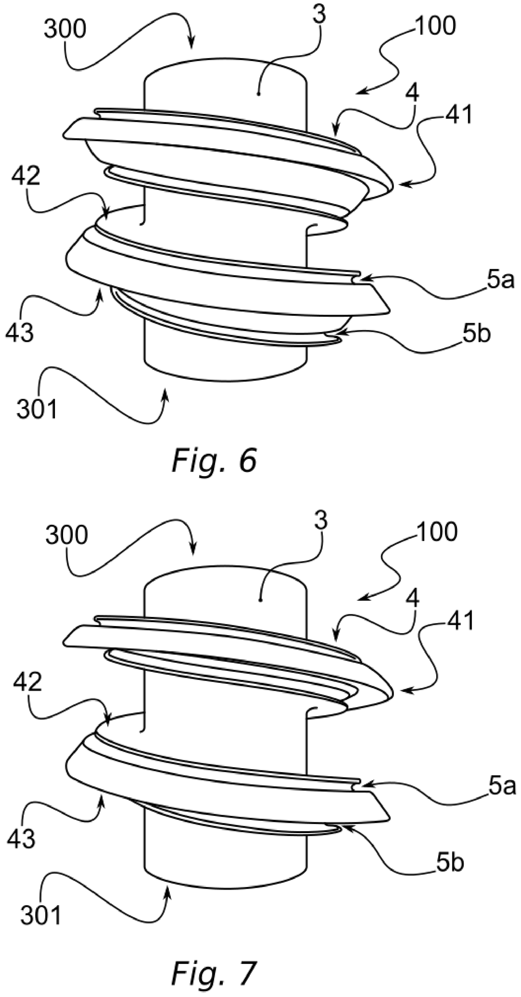

Un exemple de réalisation d'une unité 100 comprenant deux gorges 5a et 5b est visible en figure 6. En particulier, la première gorge 5a se situe à proximité de la crête 41 et la deuxième gorge 5b est située en partie inférieure du flanc aval 43. Les deux gorges 5a et 5b sont configurées pour recevoir respectivement un jonc 5a et 5b. La présence d'un jonc permet d'éviter un frottement direct entre le récipient 2 et le corps 3 ou l'hélice 40 de l'unité 100, et permet donc d'éviter les rayures ou griffures. En outre, le jonc 5a, 5b est amovible et peut être facilement remplacé en cas d'usure.

De plus, en fonction des dimensions du jonc 5a, 5b à insérer, les dimensions de la gorge correspondante 5a, 5b peuvent varier.

Un autre exemple de réalisation d'une unité 100 comprenant deux gorges 5a et 5b est visible en figure 7. En particulier, la première gorge 5a se situe sur la partie inférieure du flanc amont 42 et la deuxième gorge 5b est située sur le flanc aval 43 à proximité de la crête 41.

Les deux gorges 5a et 5b sont configurées pour recevoir respectivement un jonc 5a et 5b. Dans des modes de réalisation, l'unité 100 comprend une troisième gorge 5c sensiblement hélicoïdale au niveau du flanc aval 43, ladite gorge 5c étant apte à recevoir un troisième jonc 50c.

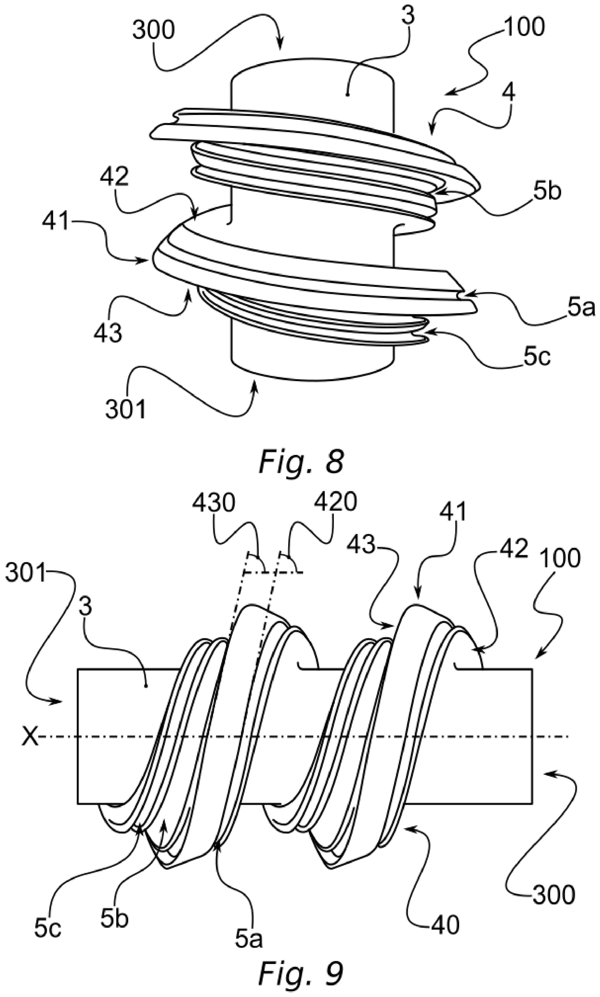

Un exemple de réalisation est visible en figure 8. Ce mode de réalisation dans lequel l'unité 100 comprend trois gorges 5a, 5b et 5c est particulièrement adapté à des récipients de type bidon de grande hauteur.

Dans des modes de réalisation, l'unité 100 est réalisée d'un seul tenant. Par exemple, l'unité 100 peut être réalisée par fabrication additive ou encore impression 3D, et en particulier par dépôt de filament fondu ou encore par fusion multijet ou par frittage sélectif par laser. De préférence, l'unité 100 est en polyamide, en particulier en nylon de type PA12. Ce mode de réalisation est particulièrement avantageux car il permet une grande flexibilité dans le dimensionnement de l'unité 100 et en particulier dans le dimensionnement des hélices 40 et des au moins deux gorges 5a, 5b. Ainsi, il est possible de produire des unités 100 de vis adaptées à tout type de récipient 2.

Dans des modes de réalisation préférés, les au moins deux gorges 5a ; 5b de l'unité 100 ont le même profil hélicoïdal que les au moins deux hélices 40 de ladite unité 100. En d'autres termes, les au moins deux gorges 5a, 5b suivent le pas p du filet 4 de vis.

Selon une caractéristique additionnel possible, dans un plan axial, l'angle amont 420 du flanc amont 42 de chaque hélice 40 est le même que l'angle aval 430 du flanc aval 43 de ladite hélice 40. L'angle de l'hélice 40 est calculé dans un plan perpendiculaire à l'axe longitudinal X du corps 3 de l'unité 100.

Il est entendu que les angles 420, 430 respectifs des au moins deux hélices 40 d'une unité 100 peuvent être différents.

De préférence, les hélices 40 d'une unité 40 présentent les mêmes angles 420, 430. Ce mode de réalisation est notamment visible en figure 9, où l'on peut voir deux hélices 40 successives présentant des angles 420, 430 d'inclinaison équivalents, et les deux angles 420 et 430 d'une même hélice 40 étant sensiblement égaux.

L'inclinaison des flancs amont 42 et aval 43 perpendiculairement à l'axe longitudinal X du corps 3 est de préférence déterminée en fonction du type de récipient 2 à traiter. Par exemple, pour un récipient 2 de grande hauteur et de faible largeur, l'inclinaison des flancs amont 42 et aval 43 doit être importante, c'est-à-dire que les angles 420 et 430 seront des angles aigus. A l'inverse, pour un récipient 2 de faible hauteur et de grande largeur, les angles 420 et 430 seront des angles obtus. En d'autres termes, la valeur des angles d'inclination 420 et 430 dépend du type de récipients 2 à traiter.

Par ailleurs, comme décrit précédemment, le corps 3 d'une unité 100 est de préférence creux. Ses dimensions sont définies notamment par son rayon r1, calculé selon un plan perpendiculaire à l'axe longitudinal X.

Dans des modes de réalisation, le rayon r1 du corps 3 est constant depuis l'extrémité amont 300 jusqu'à l'extrémité aval 301.

Selon une variante possible, le rayon r1 du corps 3 est croissant ou décroissant depuis l'extrémité amont 300 jusqu'à l'extrémité aval 301.

L'invention concerne également une vis 1 d'alimentation, ladite vis 1 s'étendant depuis une extrémité amont 30 vers une extrémité aval 31 le long d'un axe longitudinal parallèle à l'axe longitudinal X. La vis 1 selon l'invention est caractérisée en ce qu'elle comprend :
- un axe 101 central s'étendant depuis l'extrémité amont 30 vers l'extrémité aval 31,
- au moins une unité 100 telle que décrite précédemment, ledit axe 101 central étant inséré dans l'ouverture du corps 3.

Dans un mode de réalisation préféré la vis 1 comprend au moins deux unités 100 telles que décrites précédemment, lesdites unités 100 étant aboutées les unes aux autres au moyen dudit axe 101 inséré dans le corps 3 de chacune desdites au moins deux unités 100, de telle sorte que les au moins deux gorges 5a, 5b de chacune desdites au moins deux unités 100 s'étendent de manière continue depuis l'extrémité amont 30 vers l'extrémité aval 31, le long de l'axe 101 central.

Dans le cadre de l'invention, la vis 1 d'alimentation est destinée à un dispositif 6 de convoyage de récipients 2.

Ainsi, la vis 1 est de préférence réalisée par une succession d'unités 100 aboutées les unes aux autres, chaque unité 100 étant identique à l'autre. Les unités 100 de vis sont donc utilisées comme des modules de vis, la vis 1 étant modulaire. En effet, compte tenu de la vitesse de convoyage des récipients 2, de leur dimension mais aussi de la cadence de la machine de traitement, la vis 1 peut présenter une très grande longueur. Il peut donc être nécessaire d'abouter des unités 100 ensemble pour constituer une vis 1 d'alimentation.

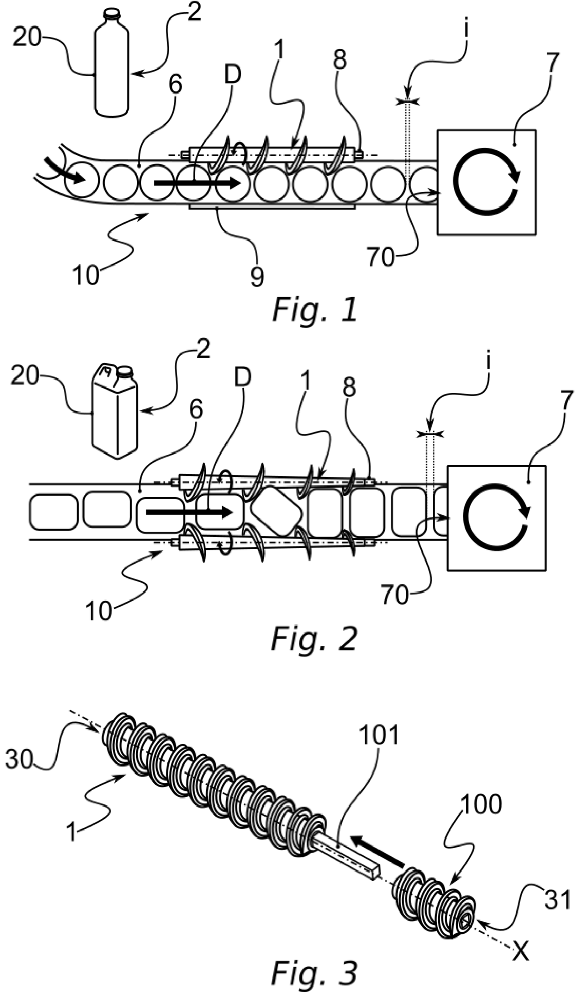

On peut voir en figure 3 un exemple de réalisation d'une telle vis 1, ladite vis comprenant une pluralité d'unités 100 aboutées les unes aux autres. Autrement dit, l'extrémité aval 301 d'une unité 100 située en amont est adjacente à l'extrémité amont 300 de l'unité 100 adjacente et située en aval, le long de l'axe 101 central.

Dans le mode de réalisation représenté en figure 3, les unités 100 présentent les mêmes caractéristiques, c'est-à-dire que chaque unité 100 comprend un filet 4 de vis présentant le même profil hélicoïdal. En d'autres termes, chaque hélice 40 de la vis 1 présente le même rayon r2 et le filet 4 présente le même pas p entre chaque hélice 40 de ladite vis 1 le long de la direction X.

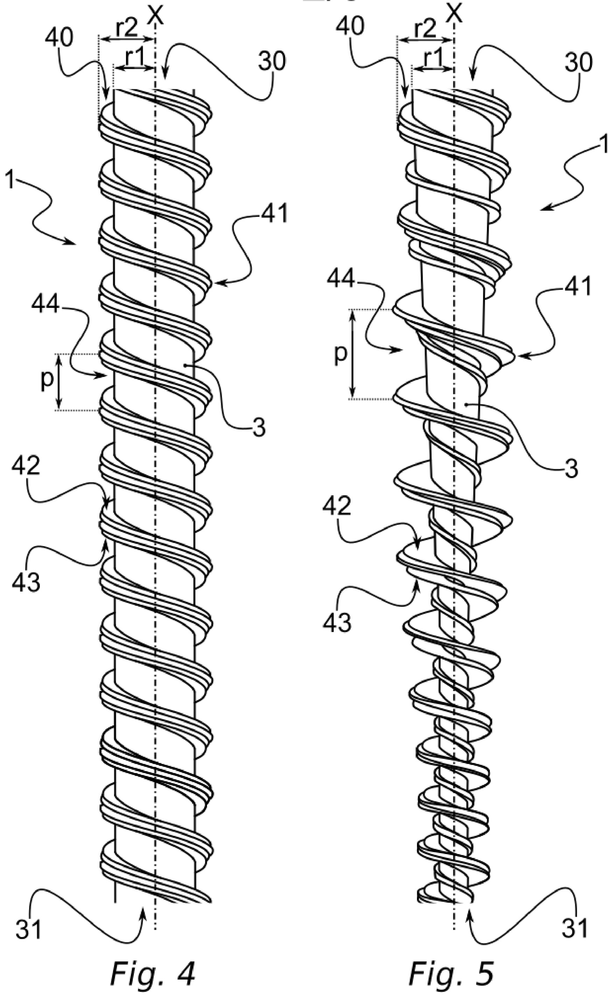

La figure 4 illustre ce mode de réalisation, où l'on peut voir que chacune des hélices 40 du filet 4 de vis présente le même rayon r2, c'est-à-dire que la crête 41 se situe à la même distance par rapport à l'axe central 101 pour chaque hélice 40. Aussi, le pas p est constant entre chaque hélice 40 depuis l'extrémité amont 30 jusqu'à l'extrémité aval 31 de la vis 1, le long de l'axe longitudinal X. Ce mode de réalisation est particulièrement adapté pour des récipients 2 de type bouteille, de forme cylindrique.

On peut voir sur la figure 3 que les unités 100 sont aboutées de sorte que les hélices 40 présentent une continuité. En d'autres termes, une unité 100 située en amont présente son extrémité 301 aval accolée à l'extrémité amont 300 de l'unité 100 adjacente située en aval, et réciproquement. De manière non représentée, les unités 100 sont aboutées de sorte que les gorges 5a, 5b forme une continuité depuis l'extrémité amont 30 jusqu'à l'extrémité aval 31, le long de l'axe 101 central.

Il est entendu que l'axe 101 central de la vis 1 est inséré de préférence dans le corps 3 d'au moins deux unités 101 de manière à ce qu'elles soient aboutées. L'axe 101 est donc parallèle à l'axe longitudinal X d'une unité 100 pour vis 1.

De manière préférée, les unités 100 aboutées les unes aux autres pour former une vis 1 présentent des caractéristiques différentes, c'est-à-dire que les hélices 40 de au moins deux unités 100 de la vis 1 présentent :
- un rayon r2 différent et/ou
- le filet 4 présente un pas p variable le long de la vis 1 le long et par rapport à l'axe 101 central.

Par rayon r2, on entend la distance entre la crête 41 de l'hélice et l'axe 101 central de la vis 1.

Par pas p, on entend la distance entre deux crêtes 41 successives.

En outre, dans des modes de réalisation, le rayon r1 du corps 3 de l'unité 100 est constant depuis l'extrémité amont 30 jusqu'à l'extrémité aval 31 de la vis 1.

Selon une variante possible, le rayon r1 du corps 3 de l'unité 100 est croissant ou décroissant depuis l'extrémité amont 30 jusqu'à l'extrémité aval 31 de la vis 1.

On comprend que, dans les cas où la vis 1 comprend au moins deux unités 100, lesdites unités 100 peuvent être aboutées de manière à ce que le rayon r1 soit constant, croissant ou décroissant, depuis l'extrémité amont 30 jusqu'à l'extrémité aval 31 de la vis 1.

Un exemple de ce mode de réalisation est illustré en figure 5. Dans cet exemple, on peut voir une vis 1 comprenant une pluralité d'unités 100, lesdites unités 100 étant aboutées de sorte que, le long de l'axe 101 central, depuis l'extrémité amont 30 jusqu'à l'extrémité aval 31 :
- les hélices 40 des unités 100 présentent des rayons r2 décroissants et
- le corps 3 des unités 100 présentent des rayons r1 décroissants.

En outre, dans cet exemple illustratif, le pas p séparant deux hélices 40 successives est également décroissant.

Ce mode de réalisation convient tout particulièrement à l'orientation de récipients 2 de type bidon, les premières hélices 40, présentant un pas p plus grand, permettant de saisir le bidon dans l'espace 44 et le rayon r2 permettant d'orienter le bidon, la vis 1 étant en rotation, dans un sens de rotation horaire ou anti-horaire, le faisant pivoter de sorte que, tout en avançant, le bidon se tourne de 90° et soit maintenu entre deux hélices 40 successives, dans un espace 44 plus étroit.

En d'autres termes, dans des modes de réalisation, le pas p entre les crêtes 41 de deux hélices 40 successives, ou encore l'espace 44 entre deux hélices 40, c'est-à-dire l'espace 44 entre la base du flanc aval de l'hélice 40 située en amont et la base du flanc amont de l'hélice 40 située en aval, diminuent entre l'extrémité amont 30 et l'extrémité aval 31 de la vis 1.

Par exemple, pour un récipient 2 de type bidon, présentant une longueur d'environ 144 millimètres (mm), une largeur d'environ 67 millimètres (mm) et une hauteur de 281 millimètres (mm), le rayon r2 est compris entre 50 et 62 millimètres (mm) et les angles amont 420 du flanc amont 42 et aval 430 du flanc aval 43 décroissent entre l'extrémité amont 30 et l'extrémité aval 31, entre 40 degrés en amont jusqu'à 15 degrés en aval. Cette décroissance de l'angle amont 420 et de l'angle aval 430 permettent un changement d'orientation du bidon en amont de la prise en charge puis une mise au pas en aval.

Dans des modes de réalisation alternatifs, le pas p entre les crêtes 41 de deux hélices 40 successives, ou encore l'espace 44 entre deux hélices 40, augmente entre l'extrémité amont 30 et l'extrémité aval 31 de la vis 1.

Dans des modes de réalisation, pour au moins une unité 100 de la vis 1, les au moins deux gorges 5a, 5b, comprennent au moins un point 51 d'inflexion dit point 51 de fixation pour la fixation de chacun des joncs 50a, 50b. De préférence, la première unité 100, située à l'extrémité amont 30, et la dernière unité 100, située à l'extrémité aval 31, comprennent chacune un point 51 d'inflexion pour jonc 50a, 50b. Ainsi, lors de l'insertion du jonc 50a, 50b respectif à la gorge 50a, 50b, ce dernier subit une déformation plastique au niveau du point 51 d'inflexion. Une fois inséré, le jonc 50a, 50b est fermement maintenue dans la gorge 5a, 5b correspondante.

Dans des modes de réalisation dans lesquels l'unité 100 comprend trois gorges 5a, 5b, 5c, pour au moins une unité 100 de la vis 1, chacune des gorges 5a, 5b, 5c comprend au moins un point 51 d'inflexion pour jonc 50a, 50b, 50c.

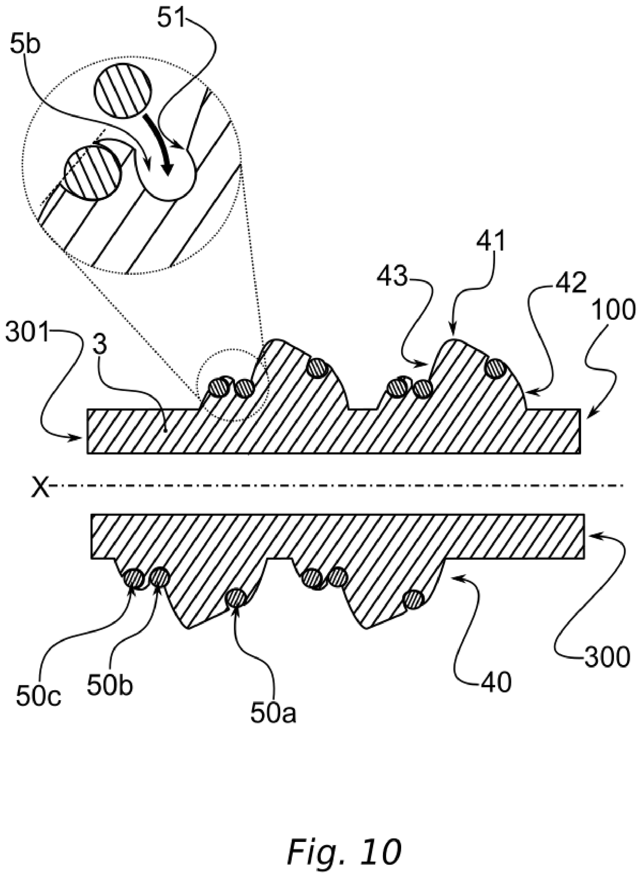

La figure 10 représente un exemple illustratif d'une unité 100 pour vis 1 selon l'invention, dans lequel les hélices 40 comprennent trois gorges 5a, 5b, 5c sensiblement hélicoïdales, dans lesquelles sont fixés respectivement un jonc 50a, 50b, 50c. Le point 51 d'inflexion est également représenté. On peut voir que les joncs 50a, 50b, 50c font saillie.

En effet, de manière préférée, une partie périphérique du jonc 50a, 50b, 50c est saillante, de manière à venir au contact d'une paroi périphérique du récipient 2 à transporter et/ou à orienter. La présence des joncs 50a, 50b, 50c permet d'éviter un frottement direct entre la vis 1 d'alimentation et le corps 30 des récipients 2.

Dans des modes de réalisation, la vis 1 comprend au moins deux joncs 50a, 50b, lesdits au moins deux joncs 50a, 50b ayant une forme complémentaire à la forme de la gorge respective 5a, 5b et s'étendant sur toute la longueur de celle-ci, c'est-à-dire depuis l'extrémité amont 30 jusqu'à l'extrémité aval 31, le long de l'axe 101 central ou encore le long de l'axe longitudinal X.

Ce mode de réalisation est particulièrement avantageux car il permet de protéger les récipients 2 lors de leur manipulation par la vis 1. Aussi, les joncs 50a, 50b sont facilement remplaçables, notamment en cas d'usure.

Dans des modes de réalisation, la vis 1 comprend trois joncs 50a, 50b et 50c. De manière préférée, les trois joncs 50a, 50b et 50c ont une forme complémentaire à la forme de la gorge respective 5a, 5b, 5c et s'étendent sur toute la longueur de celle-ci, c'est-à-dire depuis l'extrémité amont 30 jusqu'à l'extrémité aval 31, le long de l'axe 101 central ou encore le long de l'axe longitudinal X.

Selon le type de récipients 2, c'est-à-dire selon leur format, leur type, ou encore leur matériau, la vis 1 peut comprendre deux ou trois joncs, voire davantage.

Dans des modes de réalisation préférés, les au moins deux joncs 50a, 50b sont en polymère, de préférence en Polytétrafluoroéthylène.

L'invention vise encore une installation 10 de conditionnement comprenant au moins :
- un dispositif 6 de convoyage de récipients 2,
- une entrée 70 d'une machine 7 de traitement,
- au moins un arbre 8 rotatif.

L'installation 10 est caractérisée en ce qu'elle comprend au moins une vis 1 d'alimentation, de mise au pas et/ou d'orientation telle que décrite précédemment. La vis 1 selon l'invention est disposée horizontalement et s'étend depuis l'amont vers l'aval selon la direction D. En d'autres termes, l'axe 101 central de la vis 1, ou encore l'axe longitudinal X, est parallèle à la direction D de convoyage. La vis 1 est située le long du dispositif 6 de convoyage et est fixée sur un arbre 8 rotatif afin de permettre sa mise en rotation. En particulier, l'axe 101 central inséré dans la vis 1 coopère avec l'arbre 8.

Un exemple illustratif d'une telle installation 10 est visible en figure 1.

Le dispositif 6 de convoyage comprend un moyen d'entraînement et par exemple une bande de convoyage dite bande sans fin, sur laquelle reposent les récipients 2. Les récipients 2 circulent en flux unifilaire, c'est-à-dire les uns derrière les autres, sur leur fond en position verticale, et ont tous la même orientation ou non. En outre, deux récipients 2 successifs sont séparés par un intervalle i, qui correspond à la distance qui les sépare.

Les récipients 2 circulent sur ce dispositif 6 de convoyage, par exemple depuis un poste précédent ou encore depuis une table d'accumulation, vers l'entrée 70 d'une machine 7 de traitement. La machine 7 de traitement peut être tout type de machine 7 présente sur une ligne de conditionnement, par exemple une étiqueteuse, remplisseuse, ou encore un tunnel de fardelage.

L'entrée 70 de la machine 7 peut comprendre une étoile de transfert, non représentée, ladite étoile étant située en aval de la vis 1 d'alimentation et dont la rotation est synchronisée avec cette dernière.

Dans des modes de réalisation, l'installation 10 comprend, le long du dispositif 6 de convoyage, un rail 9 latéral de guidage situé du côté opposé et faisant face à la vis 1 d'alimentation, comme représenté en figure 1. Le corps 20 du récipient 2 glisse contre le rail 9 de guidage d'un côté, et est en prise avec la vis 1 de l'autre côté du dispositif 6 de convoyage.

Ainsi, les récipients 2 circulent sur le dispositif 6 de convoyage, sont guidés par un rail 9 de guidage, par exemple de type glissière, tout en étant pris en charge par la vis 1, en pénétrant au moins partiellement dans l'espace 44 séparant deux hélices 40 successives. Le profil sensiblement hélicoïdal de la vis 1 favorise la prise des récipients 2 entre les hélices 40 du filet 4. Les récipients 2 ainsi maintenus peuvent avantageusement être orientés, espacés ou rapprochés en fonction des besoins de la machine 7 de traitement.

Dans des modes de réalisation, l'installation 10 comprend une deuxième vis 1 et un deuxième arbre 8 rotatif. Comme illustré schématiquement en figure 2, la deuxième vis 1 se situe du côté opposé du dispositif 6 de convoyage, et s'étend depuis l'amont vers l'aval selon la direction D de convoyage. La deuxième vis 1 est fixée sur le deuxième arbre 8 rotatif relié au corps 3 de ladite deuxième vis 1 afin de permettre sa mise en rotation. En particulier, les deux vis 1 sont symétriques et sont configurées pour tourner dans un sens opposé.

Enfin, l'invention vise un procédé d'alimentation en récipients 2 d'une entrée 70 d'une machine 7 de traitement desdits récipients 2, le procédé comprenant au moins les étapes suivantes :
- transporter les récipients 2 sur un dispositif 6 de convoyage,
- mettre au pas et/ou orienter les récipients 2 avant leur entrée dans la machine 7 de traitement, chaque corps 20 du récipient 2 étant apte à s'engager au niveau de l'extrémité amont 31 d'une vis 1 entre deux hélices 40 formées sur le pourtour du corps 3 de ladite vis. En d'autres termes, les récipients 2 sont aptes à être en prise entre deux hélices 40 successives le long de la vis 1.

Le procédé selon l'invention est caractérisé en ce que l'étape de mise au pas et/ou d'orientation des récipients 2 est mise en œuvre au moyen d'au moins une vis 1 d'alimentation telle que décrite précédemment.

Les récipients 2 sont donc transportés sur le dispositif 6 de convoyage, et espacés en amont d'un intervalle i donné. Ils sont ensuite pris en charge par au moins une vis 1 d'alimentation, qui tourne sur elle-même. Chaque récipient 2 pénètre au moins partiellement dans un espace 44, entre deux hélices 40 successives. La vis 1 effectuant une rotation, le récipient 1 peut être accéléré ou ralenti, en fonction du différentiel de vitesse entre la vitesse de rotation de la vis 1 et le dispositif 6 de convoyage. Ce différentiel de vitesse va avoir pour effet respectivement soit d'augmenter l'intervalle i entre deux récipients 2 successifs, soit de diminuer cet intervalle i. La présence des au moins deux gorges 5a, 5b sur ladite vis 1 permet d'y insérer au moins deux joncs 50a, 50b, un jonc 50a, 50b étant placé respectivement dans chaque gorge 5a, 5b. Ces au moins deux joncs 50a, 50b permettent de protéger le corps 20 des récipients 2 lors de la manipulation desdits récipients 2 et sont facilement remplaçables en cas d'usure.

La vis 1 est également apte à effectuer un changement d'orientation d'un récipient 2. Ainsi, alors que le récipient 2 est pris en charge par la vis 1 au niveau de l'extrémité amont 30 dans une orientation donnée, il en est déchargé au niveau de l'extrémité aval 31 dans une orientation différente de celle du départ.

Le procédé selon l'invention est donc apte à être mis en œuvre au sein d'une installation 10 de conditionnement telle que décrite précédemment.

Réciproquement, l'installation 10 telle que décrite précédemment est apte à mettre en œuvre le procédé selon l'invention.

L'invention permet avantageusement de fournir une vis 1 d'alimentation apte à alimenter une machine 7 de traitement de récipients 2 de manière efficace et sans risques d'abîmer le corps 20 desdits récipients 2.

## Revendications

1. Unité (100) pour vis (1) d'alimentation, de mise au pas et/ou d' orientation de récipients (2) comprenant :
- un corps (3) central s'étendant depuis une extrémité amont (300) vers une extrémité aval (301) le long d'un axe longitudinal (X),
- un filet (4) de vis sensiblement hélicoïdal solidaire dudit corps (3), ledit filet (4) comprenant une pluralité d'hélices (40), chaque hélice (40) comprenant une crête (41), un flanc amont (42) et un flanc aval (43), ledit filet (4) étant configuré pour venir en prise entre lesdits récipients (2),
unité (100) **caractérisée en ce qu'**elle comprend pour chaque hélice (40) au moins :
- une première gorge (5a) sensiblement hélicoïdale au niveau du flanc amont (42) de ladite hélice (40) et apte à recevoir un premier jonc (50a), et
- une deuxième gorge (5b) sensiblement hélicoïdale au niveau du flanc aval (43) de ladite hélice (40) et apte à recevoir un deuxième jonc (50b).

2. Unité (100) selon la revendication précédente, **caractérisée en ce qu'**elle comprend :
- une troisième gorge (5c) sensiblement hélicoïdale au niveau du flanc aval (43) de ladite hélice (40) et apte à recevoir un troisième jonc (50c).

3. Unité (100) selon la revendication 1 ou 2, **caractérisée en ce qu'**elle est réalisée d'un seul tenant.

4. Unité (100) de vis selon la revendication précédente, **caractérisée en ce qu'**elle est réalisée en impression 3D, et de préférence en polyamide.

5. Unité (100) selon l'une quelconque des revendications précédentes, **caractérisée en ce que** les au moins deux gorges (5a, 5b) ont le même profil hélicoïdal que l'hélice (40).

6. Unité (100) selon l'une quelconque des revendications précédentes, **caractérisée en ce que**, dans un plan axial perpendiculaire à l'axe longitudinal (X) du corps (3), l'angle amont (420) du flanc amont (42) de l'hélice (40) est identique à l'angle aval (430) du flanc aval (43) de ladite hélice (40).

7. Vis (1) d'alimentation, de mise au pas et/ou d'orientation de récipients (2), ladite vis (1) s'étendant depuis une extrémité amont (30) vers une extrémité aval (31), vis (1) **caractérisée en ce qu'**elle comprend :
- un axe (101) central,
- au moins une unité (100) selon l'une quelconque des revendications précédentes, l'axe (101) étant inséré dans le corps (3) de ladite unité (100).

8. Vis (1) selon la revendication précédente, **caractérisée en ce qu'**elle comprend au moins deux unités (100), lesdites unités (100) étant aboutées les unes aux autres au moyen dudit axe (101) central inséré dans le corps (3) de chacune desdites au moins deux unités (100), de sorte que les au moins deux gorges (5a, 5b) de chacune desdites au moins deux unités (100) s'étendent de manière continue depuis l'extrémité amont (30) vers l'extrémité aval (31).

9. Vis (1) selon la revendication 7 ou 8, **caractérisée en ce que** les au moins deux gorges (5a, 5b) comprennent au moins un point (51) de fixation pour jonc (50a, 50b).

10. Vis (1) selon l'une quelconque des revendications revendication 7 à 9, **caractérisée en ce qu'**elle comprend au moins deux joncs (50a, 50b), lesdits au moins deux joncs (50a, 50b) ayant une forme complémentaire à la forme de la gorge respective (5a, 5b) et s'étendant sur toute la longueur de celle-ci.

11. Vis (1) selon la revendication précédente, **caractérisée en ce que** les au moins deux joncs (50a, 50b) sont en polymère, de préférence en Polytétrafluoroéthylène.

12. Vis (1) selon l'une quelconque des revendications 7 à 11, **caractérisée en ce que**, depuis l'extrémité amont (30) et jusqu'à l'extrémité aval (31), :
- les hélices (40) formant le filet (4) ont un rayon (r2) décroissant et **en ce que**
- le pas (p) séparant deux hélices (40) successives est décroissant.

13. Installation (10) de traitement de récipients (2) comprenant au moins :
- un dispositif (6) de convoyage desdits récipients (2),
- une entrée (70) d'une machine (7) de traitement,
- au moins un arbre (8) rotatif,
installation (10) **caractérisée en ce qu'**elle comprend au moins une vis (1) d'alimentation, de mise au pas et/ou d' orientation selon l'une quelconque des revendications 7 à 12, ladite vis (1) étant disposée horizontalement et s'étendant de l'amont vers l'aval selon une direction (D) le long d'un côté dudit dispositif (6) de convoyage, ladite au moins une vis(1) étant fixée sur ledit au moins un arbre (8) rotatif afin de permettre sa mise en rotation.

14. Installation selon la revendication précédente, **caractérisée en ce qu'**elle comprend en outre un rail (9) de guidage situé du côté opposé et le long dudit dispositif (6) de convoyage, ledit rail (9) faisant face à ladite vis (1).

15. Installation (10) de convoyage selon la revendication 13, **caractérisée en ce qu'**elle comprend un deuxième arbre (8) rotatif et une deuxième vis (1), ladite deuxième vis (1) étant située du côté opposé du dispositif (6) de convoyage et s'étendant depuis l'amont vers l'aval selon la direction (D), ladite deuxième vis (6) étant fixée sur ledit deuxième arbre (8) rotatif relié à de ladite deuxième vis (1) afin de permettre sa mise en rotation , les deux vis (1) étant symétriques et configurées pour tourner dans un sens opposé.

16. Procédé d'alimentation en récipients (2) d'une entrée (70) d'une machine (7) de traitement desdits récipients (2), ledit procédé comprenant au moins les étapes suivantes :
- transporter les récipients (2) sur un dispositif (6) de convoyage,
- mettre au pas et/ou orienter lesdits récipients (2) avant leur entrée dans la machine (7) de traitement, chaque corps (20) du récipient (2) étant apte à s'engager au niveau de l'extrémité amont (31) d'une vis (1) entre deux hélices (40) formées sur le pourtour du corps (3) de ladite vis (1),
procédé **caractérisé en ce que**
- l'étape de mise au pas et/ou d'orientation des récipients (2) est mise en œuvre au moyen d'au moins une vis (1) selon l'une quelconque des revendications 7 à 12.
